(19) Europäisches Patentamt
European Patent Office
Office européen des brevets

(11) Veröffentlichungsnummer : **0 156 263**
**B1**

(12) # EUROPÄISCHE PATENTSCHRIFT

(45) Veröffentlichungstag der Patentschrift : **12.11.86**

(21) Anmeldenummer : **85102996.7**

(22) Anmeldetag : **15.03.85**

(51) Int. Cl.⁴ : **C 07 C 69/92**, A 01 N 37/40, C 07 C 67/14, C 07 C 67/08, A 01 N 37/10, C 07 C 69/76

(54) **Neue Ester, Verfahren zu ihrer Herstellung und ihre Verwendung zur Bekämpfung von Schädlingen.**

(30) Priorität : **22.03.84 DE 3410543**

(43) Veröffentlichungstag der Anmeldung : **02.10.85 Patentblatt 85/40**

(45) Bekanntmachung des Hinweises auf die Patenterteilung : **12.11.86 Patentblatt 86/46**

(84) Benannte Vertragsstaaten : **AT BE CH DE FR GB IT LI NL**

(56) Entgegenhaltungen :
**EP-A- 0 095 117**
**DE-A- 2 512 641**
**FR-A- 2 111 543**
**US-A- 3 996 380**

(73) Patentinhaber : **BASF Aktiengesellschaft**
**Carl-Bosch-Strasse 38**
**D-6700 Ludwigshafen (DE)**

(72) Erfinder : **Hofmeister, Peter, Dr.**
**Bruesseler Ring 32**
**D-6700 Ludwigshafen (DE)**
Erfinder : **Buerstinghaus, Rainer, Dr.**
**Weinbergstrasse 85**
**D-6940 Weinheim (DE)**
Erfinder : **Adolphi, Heinrich, Dr.**
**Kalmitweg 11**
**D-6703 Limburgerhof (DE)**

**EP 0 156 263 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue spezielle Propargylester, Verfahren zu ihrer Herstellung, Schädlingsbekämpfungsmittel, die diese Ester als Wirkstoffe enthalten, sowie ein Verfahren zur Bekämpfung von Schädlingen mit diesen Wirkstoffen.

Es ist bekannt, daß Alkylester von Alkinsäuren oder Alkinylester geeignet sind zur Bekämpfung von Milben (US 4 024 278 bzw. US 3 996 380). Die Wirkung ist jedoch auf Arachniden beschränkt.

Es wurde nun gefunden, daß Ester der Formel I

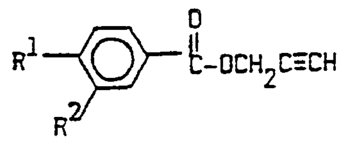

(I)

in der

$R^1$ für $OC_nH_{2n+1}$ (n = 1,2), $OCF_3$, $OCF_2CHF_2$, F und

$R^2$ für H oder mit $R^1$ zusammen für $—O(CH_2)_mO—$ (m = 1,2) steht,

insektizid, akarizid und besonders ovizid sehr gut wirksam und bekannten Wirkstoffen ähnlicher Struktur bzw. gleicher Wirkungsrichtung überlegen sind.

Die Ester der Formel I können durch Umsetzung entsprechender Säuren II mit Propargylalkohol erhalten werden (vgl. Houben-Weyl, Methoden der organ. Chemie, Band VIII, S. 516 ff, Georg-Thieme-Verlag, Stuttgart 1952).

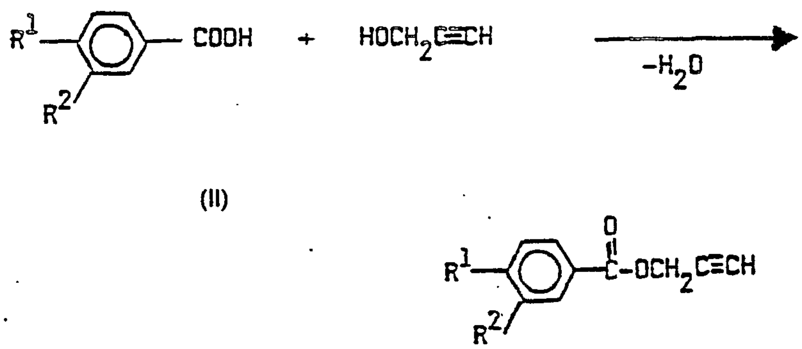


(II)

(I)

Die Umsetzung kann in an sich bekannter Weise durch Zusatz von Katalysatoren wie Schwefelsäure, Halogenwasserstoff, Sulfonsäuren, sauren Ionenaustauschern beschleunigt und das Veresterungsgleichgewicht im gewünschten Sinne verschoben werden, indem man dem Reaktionsgemisch das Wasser oder den Ester I entzieht, z. B. durch azeotrope Destillation oder durch Bindung des Wassers an Schwefel- oder Halogenwasserstoffsäure.

Ebenso gut kann man die entsprechenden Säurehalogenide III mit Propargylalkohol in Gegenwart eines Säureacceptors umsetzen (vgl. Houben-Weyl, Methoden der organ. Chemie, Band VIII, S. 541 ff, Georg-Thieme-Verlag, Stuttgart 1952).

Als Säurebindemittel eignen sich die üblichen basischen Mittel, insbesondere aliphatische aromatische und heterocyclische Amine, z. B. Triethylamin, Dimethylamin, Piperidin, Dimethylanilin, Dimethylbenzylamin und Pyridin.

Die Umsetzung kann in einem Lösungs- oder Verdünnungsmittel ausgeführt werden. Hierzu sind die angeführten Säureacceptoren selbst oder beispielsweise folgende Lösungs- oder Verdünnungsmittel oder Gemische derselben geeignet :

Aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlorethan, Chlorbenzol wie Diethyl- und Di-n-butylether, Methyl-tert.-butylether, Tetrahydrofuran, Dioxan ; Ketone beispielsweise Aceton, Methylethylketon, Methylisopropylketon ; ferner Nitrile wie Acetonitril und Propionitril.

Üblicherweise setzt man die Ausgangsstoffe in stöchiometrischem Verhältnis ein. Ein Überschuß des einen oder anderen kann in Einzelfällen aber durchaus vorteilhaft sein.

Die Umsetzung verläuft gewöhnlich oberhalb von 0 °C mit ausreichender Geschwindigkeit. Da sie meist unter Wärmeentwicklung verläuft, kann es von Vorteil sein, eine Kühlmöglichkeit vorzusehen.

Die erfindungsgemäßen Ester können außerdem noch nach praktisch allen bekannten Verfahren der Estersynthese hergestellt werden, beispielsweise durch Umsetzung von entsprechenden Säureanhydriden mit Propargylalkohol, durch Umsetzung von entsprechenden Salzen mit Propargylhalogeniden oder durch Umesterung (vgl. Houben-Weyl a.a.O. S. 508-628).

Die als Ausgangsmaterial benötigten substituierten Benzoesäuren der Formel II sind bekannt und mindestens teilweise handelsüblich ; die aus ihnen in einigen Fällen hergestellten Säurechloride wurden z. T. nach Houben-Weyl, a.a.O. S. 463-476 erhalten.

Die Herstellung aller erfindungsgemäßen Verbindungen der Formel I ist durch entsprechende Abwandlung des folgenden Beispiels möglich :

Beispiel 1

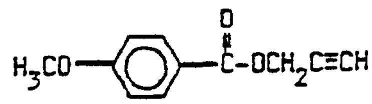

6,2 g (0,11 mol) Propargylalkohol werden in 50 ml Pyridin vorgelegt ; unter Kühlung im Eisbad tropft man 17,1 g (0,10 ml) Anissäurechlorid zu. Die Mischung wird anschließend 24 Stunden bei Raumtemperatur gerührt, dann in Eiswasser gegossen, das ausfallende Produkt abgesaugt, mit 2nHCl und Wasser gewaschen und getrocknet. Man erhält 18,1 g (95,3 % Ausbeute) eines farblosen, kristallinen Feststoffes vom Schmelzpunkt 47-48 °C.

$C_{11}H_{10}O_3$ (190)
ber. : C 69,5 H 5,3
gef. : C 69,6 H 5,5
60 MHz-$^1$H-NMR-Spektrum in $CDCl_3$ (δ-Werte in ppm).
2,55 (t, 1H) ; 3,87 (s, 3H) ; 4,93 (d, 1H) ;
6,94 (d, 2H) ; 8,05 (d, 2H).

Tabelle 1

| Beispiel | $R^1$ | $R^2$ | $^1$H-NMR-Daten (60 MHz, $CDCl_3$, δ-Werte in ppm) |
|---|---|---|---|
| 2 | $OCF_3$ | H | 2,57 (t, 1H), 4,95 (d, 2H), 7,28 (d, 2H), 8,17 (d, 2H) |
| 3 | $OCH_2CH_3$ | H | 1,43 (t, 3H), 2,56 (t, 1H), 4,09 (q, 2H), 4,89 (d, 2H), 6,88 (d, 2H), 8,01 (d, 2H) |
| 4 | $OCF_2CHF_2$ | H | 2,57 (t, 1H), 4,92 (d, 2H), 5,93 (tt, 1H), 7,27 (d, 2H), 8,10 (d, 2H) |
| 5 | F | H | 2,55 (t, 1H), 4,91 (d, 2H), 6,98-7,42 (m,2H), 7,98-8,35 (m, 2H) |
| 6 | $-OCH_2O-$ | | 2,53 (t, 1H), 4,90 (d, 1H), 6,08 (d, 2H), 6,87 (d, 1H), 7,52 (s, 1H), 7,72 (d, 2H) |

Die Propargylester der Formel I sind geeignet, Schädlinge aus der Klasse der Insekten und Spinnentiere wirksam zu bekämpfen. Sie können im Pflanzenschutz sowie auf dem Hygiene-, Vorratsschutz- und Veterinärsektor als Schädlingsbekämpfungsmittel eingesetzt werden und wirken vorzugsweise auf die Eiablage der Schädlinge.

Die vorstehend aufgeführten und andere erfindungsgemäße Wirkstoffe werden auf die für Insektizide übliche Weise angewendet. Angaben zur Formulierung, Anwendungstechnik und Wirkungsweise und Angaben über geeignete Mischungspartner zur Erzielung synergistischer und anderer vorteilhafter Wirkungen können beispielsweise der US-PS 4 320 122 entnommen werden.

Die Wirkstoffkonzentrationen in den anwendungsfertigen Zubereitungen können in größeren

Bereichen variiert werden.

Im allgemeinen liegen sie zwischen 0,000 01 und 10 %, vorzugsweise zwischen 0,001 und 0,1 %.

Die Aufwandmenge an Wirkstoff beträgt unter Freilandbedingungen 0,02 bis 10, vorzugsweise 0,1 bis 2,0 kg/ha.

Anwendungsbeispiel 1

Der im Beispiel 1 beschriebene Wirkstoff erzielt bei der Einwirkung auf Insekteneier eine hohe spezifische Wirkung ; es wurde im Tauchversuch beobachtet bei

| | | |
|---|---|---|
| Dysdercus | bei 0,01 % | 100 % Mort. |
| Prodenia | bei 0,01 % | ca. 80 % Mort. |
| Agrotis | 0,004 % | ca. 80 % Mort. |
| Heliothis | 0,004 % | ca. 80 % Mort. |
| Ephestia | 0,1 % | 100 % Mort. |
| Kartoffelkäfer | 0,02 % | 100 % Mort. |

(Prozentangabe : Wirkstoff in wäßriger Lösung).

Auch im Spritzbahn-Versuch, bei einer Aufwandmenge von 400 l/ha, trat noch deutliche Wirkung auf, und zwar bei

| | | |
|---|---|---|
| Plutella-Eiablagen | 0,1 % | ca. 90 % Mort. |
| Prodenia-Eiablagen | 0,1 % | ca. 80 % Mort. |
| Heliothis-Eiablagen | 0,04 % | ca. 90 % Mort. |
| Epilachna-Eiablagen | 0,1 % | 100 % Mort. |

Anwendungsbeispiele 2-7

Für die folgenden Anwendungsbeispiele wurden als Vergleichsmittel eingesetzt :

I

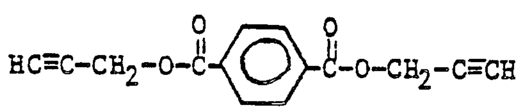

II

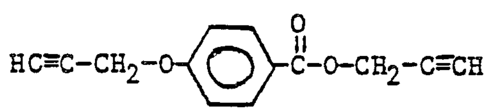

die beide in der US-PS 3 996 380 als wirksam im Sinne der Erfindung beschrieben werden.

Wirkung auf Eier der Baumwollwanze (Dysdercus intermedius)

Ca. 200 frisch abgelegte Eier der Baumwollwanze heftet man auf Klebestreifen und taucht diese in die wäßrige Wirkstoffaufbereitung. Darauf lagert man die Streifen bei 25 °C und 70 % Luftfeuchtigkeit bis zum Schlüpfen der unbehandelten Kontrolle.

Ergebnis

| Beispiel | % | % Mort. |
|---|---|---|
| 1 | 0,01 | 100 |
| 2 | 0,1 | 100 |
| 3 | 0,04 | 100 |
| 4 | 0,02 | 100 |
| 5 | 0,002 | 100 |
| 6 | 0,01 | 100 |
| Vergleichsmittel 1 | 0,1 | 40 |

Wirkung auf Eiablagen von Ostrinia nubilalis

Die weiblichen Falter legen ihre Eier in geschlossenen Gruppen auf Pergamentpapier ab. Ausgeschnittene Papierstreifen, die ca. 200-300 Eier tragen, werden 2 Tage nach der Ablage ca. 5 Sekunden in die wäßrige Wirkstoffaufbereitung getaucht. Anschließend legt man sie in eine Petrischale (Durchmesser 10 cm) auf feuchten Zellstoff.

Die Auswertung erfolgt nach Schlüpfen der Kontrolle, die nach 5 bis 6 Tagen beginnt.

Ergebnis

| Beispiel | % | % Mort. |
|---|---|---|
| 1 | 0,01 | 100 |
| 6 | 0,01 | 100 |
| Vergleichsmittel II | 0,01 | ca. 90 |

Wirkung auf Eiablagen von Prodenia litura

Die weiblichen Falter legen ihre Eier geschlossenen Gruppen auf Pergamentpapier ab. Ausgeschnittene Papierstreifen, die ca. 200-300 Eier tragen, werden 2 Tage nach der Ablage ca. 5 Sekunden in die wäßrige Wirkstoffaufbereitung getaucht. Anschließend legt man sie in eine Petrischale (Durchmesser 10 cm) auf feuchten Zellstoff.

Die Auswertung erfolgt nach Schlüpfen der Kontrolle, die nach 5 bis 6 Tagen beginnt.

Ergebnis

| Beispiel | % | % Mort. |
|---|---|---|
| 1 | 0,01 | ca. 80 |
| 4 | 0,02 | ca. 80 |
| 5 | 0,01 | ca. 90 |
| Vergleichsmittel II | 0,01 | ca. 20 |

Wirkung auf Eiablagen von Agrotis ypsilon

Die weiblichen Falter legen ihre Eier in geschlossenen Gruppen auf Pergamentpapier ab. Ausgeschnittene Papierstreifen, die ca. 200-300 Eier tragen, werden 2 Tage nach der Ablage ca. 5 Sekunden in die wäßrige Wirkstoffaufbereitung getaucht. Anschließend legt man sie in eine Petrischale (Durchmesser 10 cm) auf feuchten Zellstoff.

Die Auswertung erfolgt nach Schlüpfen der Kontrolle, die nach 5 bis 6 Tagen beginnt.

Ergebnis

| Beispiel | % | % Mort. |
|---|---|---|
| 1 | 0,004 | 80 |
| 6 | 0,004 | 100 |

Wirkung auf Eier von Heliothis virescens

2-Tage alte Eier von Heliothis virescens werden für ca. 5 Sekunden in die wäßrige Wirkstoffaufbereitung getaucht.

Anschließend legt man sie in eine Petrischale auf feuchten Zellstoff.

Die Auswertung erfolgt nach dem Schlüpfen der unbehandelten Kontrolle.

Ergebnis

| Beispiel | % | % Mort. |
|---|---|---|
| 1 | 0,01 | ca. 90 |
| 6 | 0,01 | ca. 90 |
| Vergleichsmittel II | 0,01 | ca. 40 |

Wirkung auf die Eiablage des Kartoffelkäfers Leptinotarsa decemlieata

2-Tage alte Eiablagen des Kartoffelkäfers werden mit der Blattunterlage in die wäßrige Wirkstoffaufbereitung für ca. 5 Sekunden getaucht.
Anschließend legt man sie in eine Petrischale auf feuchten Zellstoff.
Die Auswertung erfolgt nach dem Schlüpfen der unbehandelten Kontrolle.

Ergebnis

| Beispiel | % | % Mort. |
|---|---|---|
| 1 | 0,02 | 100 |
| 1 | 0,01 | ca. 90 |
| Vergleichsmittel II | 0,01 | ca. 50 |

**Patentansprüche**

1. Ester der Formel I

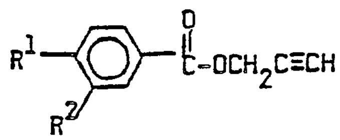

(I)

in der
$R^1$ für $OC_nH_{2n+1}$ (n = 1, 2), $OCF_3$, $OCF_2CHF_2$, F und
$R^2$ für H oder mit $R^1$ zusammen für $—O(CH_2)_mO—$ (m = 1, 2) steht.

2. Verfahren zur Herstellung der Ester der Formel I gemäß Anspruch 1, dadurch gekennzeichnet, daß man entsprechende Benzoesäuren II

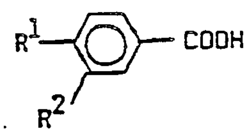

(II)

mit Propargylalkohol umsetzt oder daß man entsprechende Säurehalogenide III

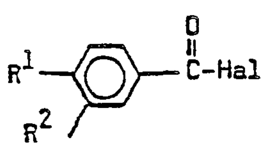

(III)

mit Propargylalkohol in Gegenwart eines Säureacceptors gegebenenfalls in Gegenwart eines Lösungs- oder Verdünnungsmittels umsetzt.

3. Schädlingsbekämpfungsmittel, enthaltend eine Verbindung gemäß Anspruch 1.

4. Schädlingsbekämpfungsmittel, enthaltend einen festen oder flüssigen Trägerstoff und mindestens einen Ester der Formel I.

5. Verwendung von Estern der Formel I gemäß Anspruch 1 als Insektizid, Akarizid, insbesondere Ovizid.

6. Verfahren zur Bekämpfung von Schädlingen, dadurch gekennzeichnet, daß man eine wirksame Menge des Esters der Formel I gemäß Anspruch 1 auf Schädlinge bzw. deren Lebensraum einwirken läßt.

**Claims**

1. An ester of the formula I

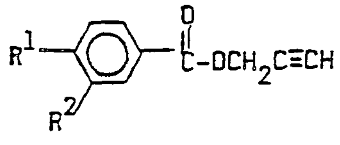

(I)

where
$R^1$ is $OC_nH_{2n+1}$ (n = 1 or 2), $OCF_3$, $OCF_2CHF_2$ or F, and
$R^2$ is H, or $R^1$ and $R^2$ together form $—O(CH_2)_mO—$ (m = 1 or 2).

2. A process for the preparation of an ester of the formula I as claimed in claim 1, wherein the corresponding benzoic acid II

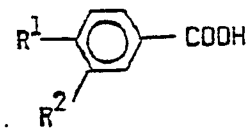

(II)

is reacted with propargyl alcohol, or the corresponding acid halide III

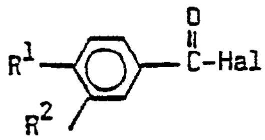

(III)

is reacted with propargyl alcohol in the presence of an acid acceptor and in the presence or absence of a solvent or diluent.

3. A pesticide containing a compound as claimed in claim 1.

4. A pesticide containing a solid or liquid carrier and at least one ester of the formula I.

5. Use of an ester of the formula I as claimed in claim 1 as an insecticide, an acaricide or, in particular, an ovicide.

6. A method of controlling pests, wherein an effective amount of an ester of the formula I as claimed in claim 1 is allowed to act on pests or their habitat.

**Revendications**

1. Esters de la formule I

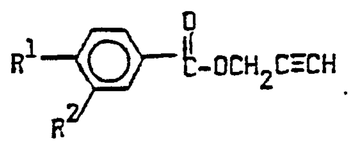

(I)

dans laquelle
$R^1$ désigne un groupe $OC_nH_{2n+1}$ (avec n = 1 ou 2), $OCF_3$, $OCF_2CHF_2$ ou F et
$R^2$ un atome d'hydrogène ou $R^1 + R^2 = —O(CH_2)_mO—$ avec m = 1 ou 2.

2. Procédé de préparation des esters de la formule I selon la revendication 1, caractérisé en ce que l'on fait réagir un acide benzoïque approprié de la formule II

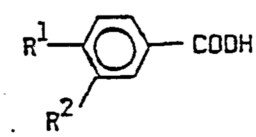

(II)

avec l'alcool propargylique ou un halogénure d'acide correspondant de la formule III

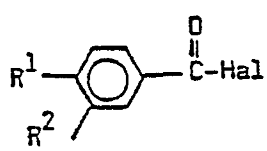

(III)

et l'alcool propargylique en présence d'un agent fixant l'acide, éventuellement dans un solvant ou diluant.

3. Composition pesticide, contenant un composé selon la revendication 1.

4. Composition pesticide, contenant au moins un ester de la formule I et une matière support solide ou liquide.

5. Utilisation d'un ester de la formule I selon la revendication 1 comme insecticide ou acaricide et en particulier comme ovicide.

6. Procédé de lutte contre des parasites, caractérisé en ce qu'on fait agir sur les parasites et (ou) sur leur biotope une quantité efficace d'un ester de la formule I selon la revendication 1.